# FASCICULE DE BREVET EUROPEEN

(11) **EP 1 978 972 B1**
(45) Date de publication et mention de la délivrance du brevet: **27.11.2013**
(21) Numéro de dépôt: 07726298.8
(22) Date de dépôt: 02.02.2007
(51) Int. Cl.: A61K 31/7028, A61P 31/00

(54) **UTILISATION D'ESTERS D'ALKYLGLUCOSIDE COMME INDUCTEURS DE BETA-DEFENSINES**
VERWENDUNG VON ALKYLGLUCOSIDESTERN ALS BETA-DEFENSIN-INDUKTOREN
USE OF ALKYLGLUCOSIDE ESTERS AS BETA-DEFENSIN INDUCERS

(30) Priorité: 02.02.2006 FR 0600949
(43) Date de publication de la demande: 15.10.2008
(73) Titulaire: Pierre Fabre Dermo-Cosmétique, 92100 Boulogne-Billancourt (FR)
(72) Inventeur: CHARVERON, Marie, F-31000 Toulouse (FR); TARROUX, Roger, F-31300 Toulouse (FR); BORDAT, Pascal, F-31320 Mervilla (FR); DUPLAN, Hélène, 31320 Auzeville Tolosane (FR)
(74) Mandataire: Regimbeau
(86) Numéro de dépôt international: PCT/EP2007/051021
(87) Numéro de publication internationale: WO 2007/088196

(56) Documents cités:
- WO-A-97/45101
- US-A- 5 837 266
- US-A- 5 888 520
- DESAI N B ET AL: "NEUE KOHLENHYDRATDERIVATE FUER DIE KOSMETIK" SOFW-JOURNAL SEIFEN, OELE, FETTE, WACHSE, VERLAG FUR CHEMISCHE INDUSTRIE, AUGSBURG, DE, vol. 117, no. 4, 14 mars 1991 (1991-03-14), pages 124-132, XP000201510 ISSN: 0942-7694
- YANG C-M ET AL: "INHIBITION OF MICROORGANISMS IN SALAD DRESSING BY SUCROSE AND METHYLGLUCOSE FATTY ACID MONOESTERS" JOURNAL OF FOOD PROCESSING AND PRESERVATION, TRUMBULL, CT, US, vol. 27, no. 4, 2003, pages 285-298, XP008068463
- LEHTONEN O-P J ET AL: "SYNERGISTIC EFFECT OF ETHYL-6-O-DECANOYL GLUCOSIDE AND CHLORHEXIDINE IN CONTACT LENS DISINFECTION" APMIS, COPENHAGEN, DK, vol. 104, no. 7/8, 1996, pages 603-606, XP008068468 ISSN: 0903-4641

## Description

La présente invention est relative à des esters d'alkylglucoside comme principes actifs provoquant un rééquilibrage de la flore commensale de la peau et des muqueuses et/ou une diminution de l'adhésion des microorganismes pathogènes sur le tissu épithélial par induction de l'expression de peptides antimicrobiens endogènes, pour son utilisation dans une composition topique dermatologique ou dermocosmétologique destinée au traitement préventif ou curatif des affections microbiennes.

Dans le cadre de la présente invention, les produits à base d'« alkyle mono- ou di-ester d'α,β-butylglucoside » sont utilisés pour déclencher une réaction immune dans un organisme hôte par génération « *in vivo* », de peptides antimicrobiens endogènes, de la famille des β-défensines (hBD-2, hBD-3) et des cathélicidines (LL-37), protecteurs vis-à-vis d'infections par des micro-organismes vivants tels les virus enveloppés, les bactéries ou parasites, conduisant ainsi à l'élimination sélective du ou des agents pathogènes. Ces produits ne stimulent pas de manière concomitante ou séparée de réactions inflammatoires, d'irritation ou d'intolérance.

Ces produits peuvent être utilisés à titre préventif (traitement prophylactique) ou curatif (traitement thérapeutique) au cours d'infections des tissus épithéliaux cutanés et cornéens, des muqueuses gingivales et vaginales, afin de rééquilibrer la microflore et favoriser l'homéostasie épithéliale au cours de phénomènes infectieux durant lesquels les processus cicatriciels sont altérés.

L'utilisation d'esters d'alkylglucoside selon la présente invention est plus particulièrement destinée à la fabrication de composition topique dermatologique ou dermo-cosmétologique destinée au traitement de la dermatite atopique, destinée à favoriser l'homéostasie épithéliale pour le traitement d'affections ophtalmiques, pour le traitement d'affections gingivales ou encore pour favoriser l'homéostasie épithéliale dans le traitement d'affections vaginales.

On connaissait des compositions dermatologiques comprenant un polysaccharide et un saccharide de faible poids moléculaire agissant en synergie, pour traiter les dermatoses de contact (US 5,837,266 et US 5,888,520).

Les interfaces épithéliales des vertébrés supérieurs ont une importance stratégique évidente puisqu'il s'agit du site de premier contact d'un organisme avec l'environnement microbien antigénique. Les épithéliums squameux stratifiés fonctionnent en tant que « barrière » vis-à-vis des traumatismes mécaniques, de la perte en eau et des infections microbiennes. Pour comprendre cette fonction, on ne peut isoler ces épithéliums de leur environnement physique, telle que l'étanchéité conférée par les systèmes d'adhésion inter-cellulaires (Presland & Dale, 2000), mais aussi chimique ou microbien.

Ainsi, l'épithélium joue parallèlement un rôle actif dans les défenses immunitaires de l'hôte, mises en jeu lors d'agressions exogènes et en particulier dans l'immunité innée ou naturelle, constituant une première ligne de défense, caractérisée par une réponse immédiate et non spécifique. Un des mécanismes est l'induction par les cellules épithéliales d'organes tels que la peau, les bronches, les intestins, les voies urinaires, l'oeil, l'appareil génital, de composés antimicrobiens de nature peptidique (Pour revue, Koczulla et Bals, 2003; Yang D., et *al* 2004). Ces peptides produits par les cellules épithéliales fonctionneraient dans une certaine mesure en maintenant la flore naturelle de micro-organismes dans un état d'équilibre propre à chaque niche écologique. La microflore commensale joue en effet un rôle important dans cet environnement en limitant la colonisation par des germes pathogènes et en stimulant les défenses naturelles immunitaires.

Ils sont répartis en quatre groupes sur la base de leur taille, de leur séquence en acides aminés et de leur structure (Koczulla & Bals, 2003). Parmi ces groupes :
■ la famille des défensines a fait l'objet d'investigations importantes depuis la fin des années 90 (pour revue Gantz, 1999; Huttner & Bevins, 1999). Toutes les défensines sont des peptides polycationiques riches en acides aminés basiques, formant trois ponts disulfures à partir de leurs six résidus cystéine aboutissant à une structure en triple feuillet bêta. Sur la base de l'alignement de ces ponts disulfures, deux classes de défensines ont été définies, les α et β-défensines (Koczulla & Bals, 2003). Principalement, quatre β-défensines ont été mises en évidence soit après isolement à partir des épithéliums, HBD-1 et 2 (Weinberg *et al,* 1998) ou par screening biomoléculaire, HBD-3 et 4 (Koczulla & Bals, 2003).
■ la famille des cathélicidines constitue un autre grand groupe de peptides antimicrobiens retrouvé chez de nombreux mammifères. Un seul peptide de cette famille a été mis en évidence chez l'être humain, hCAP18/LL-37 (Zanetti *et al,* 2004). Son expression a été retrouvée chez plusieurs tissus épithéliaux et en particulier dans les kératinocytes lors de pathologies cutanées de type inflammatoire (Frohm *et al,* 1997), mais aussi chez diverses cellules immunitaires (monocytes, cellules NK et lymphocytes B) (*Yang et al, 2004*)*.* Ce peptide est composé au départ d'un peptide signal à l'extrémité NH₂-terminale (pré-région), d'une région conservée, le domaine cathelin-like (pro-région) et du domaine antimicrobien en COOH terminal LL-37. La cathélicidine est stockée dans les granules des neutrophiles sous forme inactive Cathelin/LL-37 puis clivée par l'élastase ou la protéinase 3 pour libérer LL-37 *(Koczulla & Bals, 2003).*

Dans la plupart des épithéliums, le peptide HBD-1 est exprimé constitutivement alors que HBD-2, HBD-3 et LL-37 sont inductibles par les stimuli pro-inflammatoires comme le TNF-α ou l'IL1-β, les stimuli infectieux et le niveau de différenciation des kératinocytes cutanés (Liu *et al,* 2002 ; *Frohm et al,* 1997). Les récepteurs ou PRRs *(Gordon, 2000)* mis en jeu pour cette induction, les plus fréquemment rencontrés sont les récepteurs de l'immunité innée et en particulier les récepteurs Toll Like ou TLR (TLR2 et TLR4) et CD14 *(Chung et Dale, 2004),* mais aussi PAR-2 (Protease activated receptor) *(Chung et al, 2004)* impliquant l'activation les voies de signalisation (NF)-κB ou AP-1.

Les β-défensines et LL-37 présentent un large spectre antimicrobien : bactéries, champignons et parasites. Leur fonction microbicide repose sur leur structure amphipathique, permettant leur association avec la surface membranaire microbienne, leur agrégation et finalement la formation de pores (Gallo RL & Huttner KM., 1998 ; *Zaiou et al, 2003).* HBD-2 exerce une action bactéricide sur les souches à gram négatif, mais le plus souvent restreinte à une action bactériostatique sur les souches à gram positif. En complément, le peptide HBD-3 a une forte activité microbicide qui inclut les bactéries de type gram négatif mais aussi gram positif tel que *Staphylococcus aureus* (*S. aureus*) (Schibli *et al,* 2002). LL-37 présente non seulement une large activité microbicide dirigée contre les bactéries à gram négatif et positif (Zaiou et al, 2003), mais aussi des effets antimicrobiens synergiques avec celui des β-défensines, notamment HBD-2 qu'il rend bactéricide vis-à-vis de S. *aureus* (Ong *et al,* 2002).

En plus de cette activité anti-microbienne directe, les β-défensines et la cathélicidine LL-37 épithéliales sont capables à des concentrations sous-micromolaires d'exercer un certain nombre d'autres activités de signalisation qui aboutissent à l'initiation des défenses immunes adaptatives et ainsi de servir de lien entre immunité innée et adaptative (pour revue Yang D., 2002 et 2004). Ainsi les β-défensines HBD-2 et -3 sont capables d'émettre un signal chimiotactique qui va permettre le recrutement des cellules dendritiques immatures et des lymphocytes T mémoires, sur le site de l'infection via le récepteur spécifique CCR6 (Yang D. *et al,* 1999; pour revue Yang D. *et al,* 2004). Par ailleurs il a été démontré que HBD-2 pouvait induire la maturation de ces lymphocytes par l'intermédiaire d'un récepteur Toll-Like TLR-4 porté par les cellules dendritiques (Biragyn A. *et al,* 2002). De son côté, LL-37 possède des propriétés importantes de signalisation comme le chimiotactisme des cellules immunitaires, à des concentrations de l'ordre du nanomolaire. Ce peptide contribue ainsi au recrutement de neutrophiles, de mastocytes, de monocytes et de macrophages par l'intermédiaire du Human Formyl Peptide Receptor Like-1 (FPRL-1) (Scott et al, 2002). (Pour revue *Yang D. et al, 2004).*

D'autres fonctions lui sont attribuées telles qu'une action protectrice vis-à-vis des chocs endotoxiques en bloquant la liaison du LPS aux cellules exprimant le CD14, mais aussi son activité dans le domaine de l'angiogenèse et de la cicatrisation (Koczulla R. et al, 2003 ; Pour revue *Yang D. et al, 2004).*

Dans le cas de certaines pathologies les β-défensines et LL-37 pourraient éviter la colonisation de l'épithélium par des micro-organismes pathogènes. Ainsi, elles sont fortement exprimées au niveau de lésions psoriatiques où des surinfections par des micro-organismes ne sont que très rarement détectées. Au contraire, dans le cas d'autres pathologies comme la dermatite atopique (DA), fréquemment accompagnée de surinfections par *S. aureus* par exemple, ces peptides ne sont pas exprimés (Ong *et al,* 2002). Ainsi, l'absence ou l'inactivation de l'expression de ces peptides peut mener à des surinfections au décours de pathologies épithéliales diverses. Dans ce cas l'expression endogène des peptides HBD-2, HBD-3 et LL-37 étant potentiellement inductible au niveau de divers épithéliums (cutané, gingival, nasal, vaginal, tractus respiratoire...) *(Kao et al, 2004; Krisanaprakornkit et al, 2002),* la stimulation locale de leur synthèse se définit comme une cible potentielle de l'induction de l'immunité innée pour apporter une réponse thérapeutique en relation avec l'écosystème microbien et l'homéostasie épithéliale.

Chez les individus sains, la surface épithéliale de la cavité orale est régulièrement colonisée par différentes souches de micro-organismes commensales *(Fusobacterium nucleatum, F. nucleatum)* sans qu'elle ne soit pour autant infectée par des micro-organismes pathogènes. La barrière mécanique, les composants de l'immunité adaptative et de l'immunité innée tels que les peptides antimicrobiens sont responsables du maintien de la balance écologique et de l'homéostasie épithéliale buccale *(Chung WO. and Dale BA, 2004)* .Le spectre antimicrobien des peptides et protéines sécrétés par l'épithélium oral et les glandes salivaires, leur régulation et les conditions de leur production, définissent la microflore de la cavité orale (Yoshimura A. *et al,* 2002). L'absence ou l'inactivation de ces peptides sont fréquemment corrélés à l'apparition de pathologies parodontales comme les parodontites, faisant suite à la colonisation par des pathogènes anaérobies à gram négatifs libérant de nombreux facteurs de virulence, tels que *Prevotella intermedia (P. intermedia) Porphyromonas gingivalis* (*P. gingivalis*) et *Actinobacillus actinomycetemcomitans (A. actinomycetemcomitans),* ou des infections opportunistes comme les candidoses (Feucht EC., et al, 2003).

Par ailleurs, ces peptides fonctionneraient dans une certaine mesure en maintenant la flore naturelle de micro-organismes dans un état d'équilibre propre à chaque niche écologique. La microflore commensale joue en effet un rôle important dans cet environnement en limitant la colonisation par des germes pathogènes et en stimulant les défenses naturelles immunitaires.

En tant que molécules de signalisation, ils pourraient stimuler la réponse adaptative après avoir recruté les cellules immunitaires au niveau cutané. Ainsi, la Demanderesse a considéré que le potentiel adjuvant indirect des « alkyle mono- ou di-ester d'α,β-butylglucoside », via la stimulation de l'expression des peptides antimicrobiens cationiques (hBD-2, hBD-3, et LL-37) au niveau de différents épithéliums (cutané, gingival, vaginal ou oculaire) peut être ciblé en vue d'un renforcement préventif du système immunitaire des individus.

L'intérêt de la Demanderesse s'est porté sur l'étude de l'effet inducteur des « alkyles mono- ou di-ester d'α,β-butylglucosides » sur l'expression des gènes codant pour les peptides cationiques antimicrobiens humains hBD-2, hBD-3 et LL-37 en comparaison de l'effet de cytokines pro-inflammatoires ou d'éléments infectieux, au niveau de modèles cellulaires (lignée de kératinocytes humains HaCat, primoculture de kératinocytes humains) et de modèles tissulaires (explants cutanés ou gingivaux, épithélium gingival reconstruit, maintenus en survie *in vitro).* Cet effet régulateur a été mis en évidence au niveau des acides ribonucléiques messagers (ARNm) de manière quantitative par la technique de transcription inverse (RT) couplée à la technique de réaction de polymérisation en chaîne (PCR) en temps réel. En complément, la synthèse peptidique induite a été qualitativement évaluée par immuno-histochimie dirigée contre les peptides hBD-2, hBD-3 et LL-37. Dans un second temps, nous avons analysé l'effet biologique *ex vivo* des « alkyle mono- ou di-ester d'α,β-butylglucoside » sur l'adhésion d'une surface cutanée par S. *aureus* à l'aide d'un modèle à double compartiment séparé.

Conformément à la présente invention, on utilisera plus particulièrement l'ester d'alkylglucoside constitué par un C₈-C₂₀-alkyle mono- ou di-ester de C₁-C₆-alkylglucoside, en particulier un C₈-C₂₀-alkyle mono- ou di-ester de butylglucoside, le fragment ester pouvant être avantageusement un mono- ou di-caprylate, -caprate, -laurate, -palmitate, -myristate, - stéarate, -cocoate d'α-butylglucoside.

Les alkylglucosides avec une chaîne alkyle formée de 8 à 20 atomes de carbone sont des agents tensioactifs dont les structures et synthèses chimiques ont été décrites dans la demande de brevet WO 93/04185.

Il est connu de WO 97/45101 que les esters d'α-alkylglucoside peuvent être utilisés comme agent anti-microbien et/ou anti-fongique pour agir en tant que conservateur dans une composition, pour la préserver d'un développement microbien. D'autre part, les propriétés moussantes des esters de méthyl glucose sont décrites dans Desai et al (« Sofw Journal Seife, Oele, Fette, Wachse, Verlag für chemische Industrie » 1991).
- Modèle cellulaire : lignée cellulaire HaCat, kératynocytes d'origine humaine, primoculture de kératinocytes humains
- Modèle tissulaire :
   - Explants de peau normale provenant de déchet opératoire de chirurgie esthétique
   - Explants de gencive normale provenant de déchet opératoire de chirurgie implantatoire ou d'épithélium gingival reconstruit à partir de cellules épithéliales orales primaires

   ➢ Les cellules HaCat sont ensemencées et cultivées en plaque 6 puits à 200 000 cellules/puits sous un volume de 2 ml de milieu de culture DMEM 1640 : (Ref: Invitrogen) complémenté par 10 % de sérum de veau foetal (SVF, Ref : CH30160.02 Perbio-Hyclone) pendant 18 h (sur la nuit).
   ➢ A T0 les cellules sont lavées avec du tampon Phosphate (PBS, Ref: 52100-039 Invitrogen) puis privées en sérum pour les rendre quiescentes pendant 24 h (ralentissement de leur croissance et élimination de toute trace de facteurs susceptibles de perturber les effets induits par les traitements ultérieurs).
   ➢ A T0+24 h les cellules sont traitées par des cytokines pro-inflammatoires (TNF-α à 10 ng/ml, IL1-β à 10 ng/ml) ou des éléments bactériens (LPS à 50 ng/ml, SEB à 100 ng/ml) ou des actifs potentiels de type « alkyle mono- ou di-ester d'α,β-butylglucoside » dilués dans du DMEM (1 ml/puits); La durée du traitement est de 3 h à 37°C.
   ➢ Les kératinocytes primaires sont préparés à partir de peau normale provenant de déchet opératoire de chirurgie esthétique. Ils sont ensemencés et cultivés en plaque 24 puits à 60 000 cellules/puits sous un volume de 0,5 ml de milieu de culture KSFM (Ref : 37010 Invitrogen) complémenté par de l'EGF (Epidermal Growth Factor) à 1,5 ng/ml et du BPE (bovine pituitary extract) à 25 µg/ml pendant 24 h.
   ➢ A J0, le milieu est remplacé par 0,5 ml de milieu KSFM complet, additionné de 1,2 nM de calcium.
   ➢ A J+3, les cellules sont traitées par des cytokines pro-inflammatoires TNF-α et IL 1-β à 10 ng/ml ou des actifs potentiels de type « alkyle mono- ou di-ester d'α,β-butylglucoside » dilués dans 0,3 ml de milieu KSFM complet, en présence de 1,2 nM de calcium. La durée du traitement est de 3 h à 37°C.
   ➢ Après plusieurs lavages dans du PBS sans antibiotiques, des punchs de 6 mm de diamètre sont préparés et disposés au fond d'un puits d'une plaque 12 puits.
   ➢ Un traitement systémique du tissu cutané affleurant non immergé est effectué par des cytokines pro-inflammatoires (TNF-αà 500ng/ml, IL1-β à 100 ng/ml) ou des actifs potentiels de type « alkyle mono- ou di-ester d'α,β-butylglucoside », dilués dans du DMEM (350 µl/puits). Les temps de stimulation sont variables de 1 h, 1 h, 15 h ou 24 h.
   ➢ Dans le cas de traitement topique, un anneau d'aluminium de 10 mm de diamètre est placé sur l'explant tissulaire afin de délimiter une surface épithéliale cutanée précise sur laquelle l'application topique sera effectuée. Avant l'application topique du traitement, une rupture de barrière cutanée réversible est effectuée à l'aide d'un solvant, l'acétone pendant 3 min. Les produits dilués dans du DMEM sont ensuite appliqués pour une incubation de 2, 5 ou 16 h, en parallèle d'un témoin de stimulation d'origine bactérienne (SEB à 50 µg/ml). Après incubation, les explants sont rincés dans le PBS et sont soit congelés rapidement dans de la résine Tissue Tek® (SAKURA, Zoeterwoude) dans l'azote liquide (-196°C) en vue de faire des cryocoupes, soit conservés à -80°C pour réaliser des extractions d'ARN totaux.

Les explants gingivaux humains de taille variable et petite sont rincés dix fois dans du PBS sans antibiotiques puis mis en culture sur une grille afin d'éviter leur immersion totale, posés côté derme. L'incubation systémique se fait dans un milieu de survie dans du DMEM seul ou en présence d'αβ-LBG brut à 10 µg/ml, à 37°C et 5 % de CO₂.

Les inserts d'épithélium gingival reconstruit sont conservés dans du milieu de maintenance (1 ml) dans des boites de culture cellulaire 6 puits pendant 24 h à 37°C et 5 % de CO₂ selon les recommandations du fournisseur SkinEthic laboratories (Nice, France). Après 24 h, le tissu est stimulé en topique par 500 µl de milieu de maintenance seul ou additionné d'αβ-LBG brut à 10 µg/ml, à 37°C et 5 % de CO₂. Du milieu de maintenance est ajouté en dessous de l'insert (1 ml) pendant la stimulation. Après découpe d'un punch de 3 mm de diamètre et son inclusion dans la résine Tissue Tek®, le reste du tissu est conservé à -80°C en vue d'une extraction des ARN totaux.

Toutes les manipulations concernant les ARN sont faites avec de l'eau, des tampons et du matériel consommable dépourvus d'ARNases
➢A partir des cellules :
   A l'arrêt du traitement, les cellules sont lavées rapidement avec du PBS et reprises dans le tampon de lyse du kit d'extraction Qiagen Rneasy^{®}. Le protocole d'extraction des ARN totaux est réalisé avec le kit selon les recommandations du fournisseur (Ref: 74106 Qiagen). Les ARN sont repris dans de l'eau sans RNAse (Ref : 0685101 Eppendorf) et conservés à -80°C.
➢ A partir des explants tissulaires :
   A l'arrêt du traitement, les explants sont lavés rapidement au PBS, égouttés et transférés dans un microtube à centrifuger sans RNAse de 1,5 ml (Eppendorf), lui-même plongé dans l'azote liquide pour congélation et conservation à -80°C. Une première étape consiste à broyer le tissu à froid mécaniquement à l'aide d'un mortier et d'un pilon en Inox (Société Pasquet, Toulouse) pour obtenir une fine poudre. Le broyat est ensuite repris dans le tampon de lyse du kit d'extraction Qiagen Rneasy^{®} et l'ARN total est extrait selon les recommandations du fournisseur. Les ARN sont repris dans de l'eau sans RNAse et conservés à -80°C.
➢ Dosage des ARN totaux :
   Il est effectué à l'aide du kit RNA 6000 Nano LabChip® (Ref : 5065-4476 Agilent Technologies) sur le Bioanalyser 2100 (Agilent Technologies) selon les indications du fournisseur. L'analyse des données est effectuée à l'aide du logiciel d'analyse « Agilent Bioanalyzer 2100 software screen » (Agilent Technologies). Ce kit permet de quantifier et de contrôler le degré de pureté des ARN vis-à-vis de l'ADN génomique et des protéines. Chaque échantillon est déposé dans un puits sur une puce (12 par puce). Les ARN totaux sont séparés par électrophorèse et visualisés grâce à un agent intercalant fluorescent. Dans notre cas, la visualisation des ARN 18S et 28S eucaryotes et le rapport ARN 28S/ARN 18S permet d'estimer la non dégradation des ARN.
➢1 µg d'ARN totaux est utilisé par échantillon afin d'être transformé en ADN complémentaire (utilisable pour la Réaction de Polymérisation en chaîne ou PCR) selon le protocole de transcription inverse ou RT. Les ARN sont dénaturés 2 min à 95°C puis le mélange de RT contenant l'enzyme de transcription inverse (AMV Reverse Transcriptase), les amorces oligo-dT, les nucléotides et le tampon sont ajoutés. Ces produits sont issus du kit Access RT-PCR Core Reagents (Ref A3500, Promega). La RT est réalisée à 42°C pendant 60 min puis l'enzyme est dénaturée à 95°C pendant 2 min. Les ADNc obtenus sont conservés à -20°C.
➢Les analyses de PCR en temps réel sont effectuées à l'aide du thermocycler à fluorescence lcycler iQ (Biorad). Cette technique permet de visualiser cycle après cycle l'accumulation de produit de PCR marqué à la fluorescence. Les ADNc correspondant à 1 µg d'ARN servent de matrice dans un mélange réactionnel de 26 µl contenant le mélange de PCR iQ™ SYBR® Green SuperMix (Ref: 170-8882, Biorad) et 0,3 µM de chaque amorce.

En complément des échantillons, un témoin sans matrice d'ADNc est déposé sur une plaque 96 puits. Pour chaque échantillon, l'amplification des différents gènes d'intérêt, hBD-2, hBD-3 et LL-37 est réalisée en duplicate en plaque 96 puits ainsi que l'amplification parallèle de trois gènes de référence choisis pour leur stabilité durant les différents traitements. Les gènes de référence utilisés sont ceux codant pour YWHAZ (Tyrosine 3-monooxygenase/tryptophan 5-monooxygenase activation protein, zeta polypeptide), GAPDH (Glyceraldehyde-3-phosphate dehydrogenase), et HPRT (human hypoxantine ribosyl transferase) ou β₂-M (Human β₂-microglobuline) (tableau 1). Les séquences des amorces utilisées pour l'amplification de ces trois gènes par PCR en temps réel sont décrites par Vandesompele J. *et al* (2002).

**Tableau 1 : Séquences des primers utilisés pour l'amplification des gènes de références par PCR en temps réel.**

| **Gènes de références** | | **Amorces** |
|---|---|---|
| human hypoxantine ribosyl transferase | **HPRT** | *Sens* 5'-TGA CAC TGG CAA AAC AAT GGA-3' |
| | | Antisens 5'- GGT CCT TTT CAC CAG CAA GCT -3' |
| Human glyceraldehyde-3-phosphate dehydrogenase | **GAPDH** | *Sens* 5'-TGCACCACCAACTGCTTA-3' |
| | | *Antisens* 5'-GGC ATG GAC TGT GGT CAT GAG-3' |
| Human β₂-microglobuline | **β₂**-**M** | *Sens* 5'-ACC CCC ACT GAA AAA GAT GA-3' |
| | | *Antisens* 5'-ATC TTC AAA CCT CCA TGA TG-3' |
| tyrosine-3-mono-oxygenase, tryptophane-5-mono-oxygenase activation protein zeta polypeptide | **YWHAZ** | *Sens* 5'-ACT TTT GGT ACA TTG TGG CTT CAA-3' |
| | | *Antisens* 5'-CCG CCA GGA CAA ACC AGT AT-3' |

La plaque est incubée dans le thermocycler pour les différentes étapes de PCR. Pendant l'amplification, l'émission de fluorescence est enregistrée à la fin de chaque cycle et analysée par le logiciel iCycler (version 3.0 BioRad). L'augmentation de l'émission de fluorescence est proportionnelle à la quantité de produits de PCR accumulée, qui est en fait proportionnelle à la quantité d'ARNm correspondant à la séquence amplifiée. Les résultats sont évalués avec le logiciel iCycler IQ optical system (Version 3.0, BioRad). Pour chaque couple d'amorce, l'efficacité de PCR est vérifiée.

Les séquences des amorces pour l'amplification des ARNm de hBD-2, hBD-3 and LL-37 ont été dessinées avec le logiciel Beacon Designer Software (version 2 .1, Biorad).

**Tableau 2 : Séquences des primers utilisés pour l'amplification des gènes hBD-2, hBD-3 et LL-37 en PCR en temps réel**

| **Gènes d'intérêt** | | **Amorces** |
|---|---|---|
| human beta-défensine 2 | **HBD-2** | *Sens* 5'-CCA GCC ATC AGC CAT GAG GGT-3' |
| | | *Antisens* 5'-GGA GCC CTT TCT GAA TCC GCA-3' |
| human beta-défensine 3 | **HBD-3** | *Sens* 5'-TAG GGA GCT CTG CCT TAC CA-3' |
| | | *Antisens* 5'-CAC GCT GAG ACT GGA TGA AA-3' |
| Human cathelicidin | **LL-37** | Sens 5'-CCA AGC AGT CAC CAG AGG ATT G-3' |
| | | Antisens 5'-GGC CTG GTT GAG GGT CAC T-3' |

Pour chaque échantillon, le niveau d'expression de chaque gène d'intérêt (hBD-2, hBD-3 et LL-37) est ensuite normalisé par rapport au niveau d'expression des gènes de référence non induits par les différents traitements à l'aide du logiciel Genorm version 3.2c (Vandesompele et al., 2002).

Après normalisation, l'induction relative au niveau transcriptionnel (ARNm) des gènes codant pour les β-défensines hBD-2 et hBD-3 et la cathélicidine LL-37 est donnée par rapport à un témoin absolu non induit (milieu seul).

Les échantillons inclus dans la résine Tissue Tek® sont coupés à froid (température de la chambre à -25°C et la température du porte-objet à - 28°C) à l'aide d'un cryostat (Leica CM 3050s, Nussloch). Les cryocoupes d'une épaisseur de 5 µm sont récupérées sur des lames porte-objet prétraitées, puis mises à sécher environ 2 h à température ambiante et stockées à -20°C pour une utilisation ultérieure. Avant de réaliser l'immuno-marquage, les coupes sont fixées à l'acétone à -20°C pendant 10 min, séchées et conservées à -20°C. Lors d'une première étape, les coupes sont hydratées puis incubées avec les anticorps primaires dirigés contre les peptides d'intérêt dilués dans du PBS. Les anticorps primaires utilisés sont les suivants :
- Goat Anti-human BD-2 (500-P161G, Peprotech Inc) dilué au 1/50^{e}
- Rabbit Anti-human defensin-3 (AB3478, Chemicon international) dilué au 1/1000^{e}
- Rabbit Anti-human LL37 (G-075-06, Phoenix Pharmaceuticals Inc) dilué au 1/50^{e}

Pour révéler le complexe Antigène-Anticorps, le kit DAKO LSAB® (DAKO Corporation) est utilisé selon les recommandations du fournisseur. La révélation est réalisée à l'aide d'un substrat de la péroxydase : la 3,3'diaminobenzidine (DAB) (DAKO Corporation). La réaction enzymatique est révélée par une coloration brune.
➢L'adhésion des micro-organismes est réalisée dans une cellule à double compartimentation, de type cellule de Franz modifiée.
➢Une suspension de *S.aureus* (CIP 53.154) équivalente à 0,5.10⁷ micro-organismes est introduite au sein du compartiment supérieur de la cellule. Le dispositif est incubé de 2 h à 37°C sous une atmosphère à 5 % de CO₂. A la fin de cette période d'incubation, le surnageant est retiré afin d'éliminer les bactéries non adhérentes. Les échantillons de peau sont ensuite rincés deux fois avec 1 ml de mélange DMEM/HAMF12. Un dénombrement bactérien à l'issue de cette étape donne la proportion de bactéries ayant adhéré à l'explant (T+2 h). Un témoin est réalisé dans le but de déterminer l'adhésion non spécifique des bactéries.
➢La stimulation de l'expression des β-défensines cutanées précède l'étape d'adhésion. Son objectif est d'étudier l'impact de l'induction des peptides antimicrobiens par des actifs potentiels de type « alkyle mono- ou di-ester d'α,β-butylglucoside » sur l'adhésion de souches bactériennes. Le traitement est effectué en topique ou en systémique. Le temps de stimulation des explants cutanés est de 24 h à 37°C en présence de 5 % de CO₂.

Pour analyse, les explants sont retirés de leur cellule, des punchs de diamètres différents (3 mm, 4 mm et 6 mm) sont effectués pour un test MTT de survie, les marquages immuno-histologiques et les analyses des transcrits par RT-PCR. Le reste de peau est utilisé pour une estimation de la flore bactérienne adhérente.

Pour la plupart des préparations testées la concentration limite à partir de laquelle une cytotoxicité des préparations d'alkyle mono- ou di-ester d'α,β-butylglucoside a pu être mise en évidence sur la lignée de kératinocytes HaCat par la méthode du rouge neutre après 24 h d'incubation est de 1,10-2 % soit 100 µg/ml.

Aux concentrations efficaces pour l'induction de l'expression des peptides hBD-2 et hBD-3, les alkyles mono- ou di-esters d'α,β-butylglucoside n'entraînent pas une augmentation de la libération de métabolites pro-inflammatoires dérivés de l'acide arachidonique, par les cellules épithéliales cutanées (lignée kératinocytaire HaCat). Ces préparations ne sont donc pas considérées comme pro-inflammatoires.

L'induction de l'expression du gène hBD-2 a été quantifiée au niveau des ARNm spécifiques transcrits par la lignée de kératinocytes humains HaCat, normalisée par rapport à l'expression constitutive de trois gènes de référence stables (HPRT, GAPDH, YWHAZ) dans les conditions de traitement testées, citées dans le tableau 3. La méthode de RT-PCR quantitative utilisée est décrite dans le matériel et méthode. Le niveau d'induction est calculé par rapport au témoin non stimulé traité dans les mêmes conditions si ce n'est l'agent stimulant.

**Tableau 3: Activité des αβ-LBG sur l'induction de l'expression du gène codant pour hBD-2 chez les cellules HaCat après 3 h de traitement : quantification des ARNm spécifiques par PCR en temps réel et détermination d'un niveau d'induction normalisé, par rapport au témoin cellulaire non stimulé.**

| Traitements | | Facteur d'induction (FI) |
|---|---|---|
| Témoin non stimulé | | 1 |
| TNF-α 10 ng/ml | | 2,5 |
| Produits de départ | αβ-butylglucoside 10 - 1 - 0,1 µg/ml | 1-1-0,6 |
| | Acide Laurique 10µg/ml | 1,1 |
| αβ-LBG brut 10 µg/ml | | 1,7 |

L'ester de laurate, αβ-LBG brut à 10 µg/ml pur à 48,6 % dont 90 % de la forme α-LBG et 10 % de forme β-LBG est actif sur l'induction de la synthèse du peptide hBD-2 au niveau transcriptionnel avec un facteur d'induction (FI) de 1,7 par rapport au témoin non stimulé, en comparaison d'un facteur 2,5 pour le TNF-α. Il a été vérifié parallèlement que les produits de départ αβ-butylglucoside et acide laurique utilisés lors de la synthèse enzymatique de cet agent ne provoquent pas une induction de la synthèse du peptide hBD-2, puisque 5,6 % Ac. laurique résiduels sont encore retrouvés dans cette préparation.

L'induction de l'expression du gène hBD-2, a été quantifiée par RT-PCR quantitative au niveau des ARNm spécifiques transcrits par des lignées primaires de kératinocytes humains issus de deux donneurs différents. Les résultats sont normalisés par rapport à l'expression constitutive de trois gènes de référence stables (HPRT, GAPDH, YWHAZ) dans les conditions de traitement testées. Le niveau d'induction est calculé par rapport au témoin non stimulé traité dans les mêmes conditions si ce n'est l'agent stimulant.

A la concentration de 0,1 µg/ml, l'ester de laurate de butylglucoside pur à 90 %, constitué uniquement de l'isoforme β présente une meilleure activité d'induction de l'expression du gène hBD-2 (FI à 2,3) que l'ester de laurate αβ-LBG pur à 90 % constitué de 5 % de l'isoforme α et 44 % de l'isoforme β (FI à 1,4) et l'ester de laurate pur à 90 %, constitué uniquement de l'isoforme α (FI à 1,2)

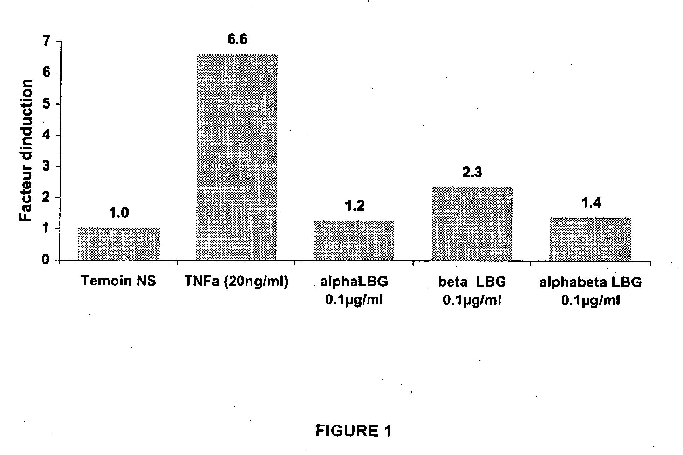

La figure 1 annexée illustre l'activité des différentes isoformes de LBG sur l'induction de l'expression du gène hBD-2 chez les kératinocytes primaires humains après 3 h de traitement : quantification des ARNm spécifiques par PCR quantitative en temps réel et détermination d'un niveau d'induction normalisé, par rapport au témoin cellulaire non stimulé (témoin NS).

Les cellules primaires NHK apparaissent plus sensibles à la stimulation par les αβ-LBG. Dès 0,1 µg/ml une induction du gène hBD-2 apparaît significative alors que 10 µg/ml sont nécessaires pour obtenir une réponse de la lignée HaCat

Le potentiel inducteur de l'ester de laurate, αβ-LBG brut a été analysé sur explant cutané par addition du traitement dans le milieu de culture, « voie systémique », ou après application topique de l'agent, « voie topique ».

### Traitement par voie systémique

➢L'induction de l'expression du gène hBD-2 a été quantifiée au niveau des ARNm spécifiques transcrits par les cellules épithéliales cutanées au sein d'explants de peau incubés, de manière systémique en présence de TNFα à 100 ng/ml, d'αβ-LBG brut à 3 et 30 µg/ml. Les variations d'expression du gène hBD-2 ont été normalisées par rapport à l'expression constitutive de trois gènes de référence stables (β-2M, GAPDH, YWHAZ). Le niveau d'induction est calculé par rapport au témoin tissulaire non stimulé traité dans les mêmes conditions si ce n'est l'agent stimulant.

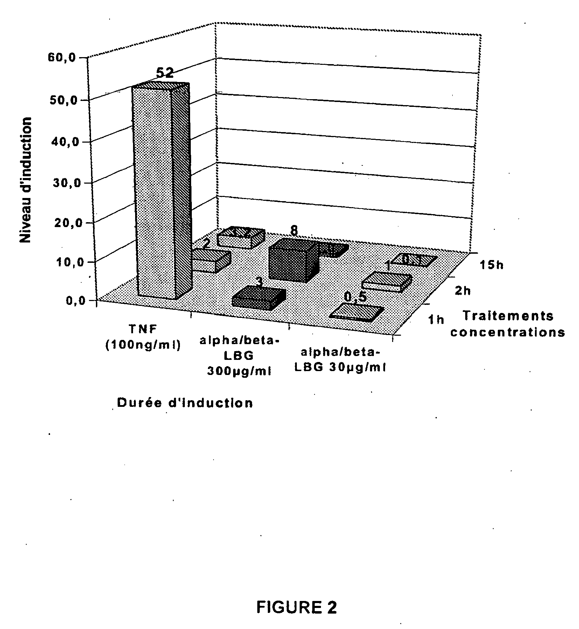

La figure 2 annexée illustre la cinétique d'induction de l'expression du gène hBD-2 au sein d'un explant cutané par l'α,β-LBG brut à différentes concentrations, par voie systémique : quantification de la synthèse des transcrits hBD-2 par RT-PCR en temps réel.

Comme pour le modèle cellulaire, nous montrons que cet agent sous forme brute à 30µg/ml est capable d'induire la synthèse des transcrits hBD-2 au niveau de l'épithélium cutané après une incubation avec la peau par voie systémique.

De plus l'étude cinétique montre que cette induction est rapidement effective, dès 1 h d'incubation. Le pic d'induction du TNFα est un peu plus précoce à 1 h de stimulation.
➢L'induction de l'expression des gènes HBD-3 et LL-37 au niveau d'un explant cutané après traitement par voie systémique pendant 2 ou 24 h par l'αβ-LBG brut à 30 µg/ml a été quantifiée au niveau des transcrits des deux peptides.

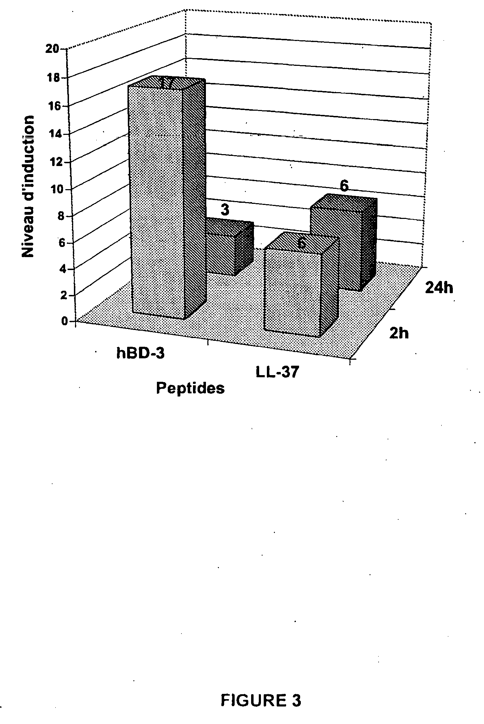

La figure 3 annexée illustre la cinétique d'induction de l'expression des gènes codant pour hBD-3 et LL-37 au sein d'un explant cutané, par l'α,β-LBG brut à différentes concentrations par voie systémique: quantification de la synthèse des transcrits par RT-PCR quantitative.

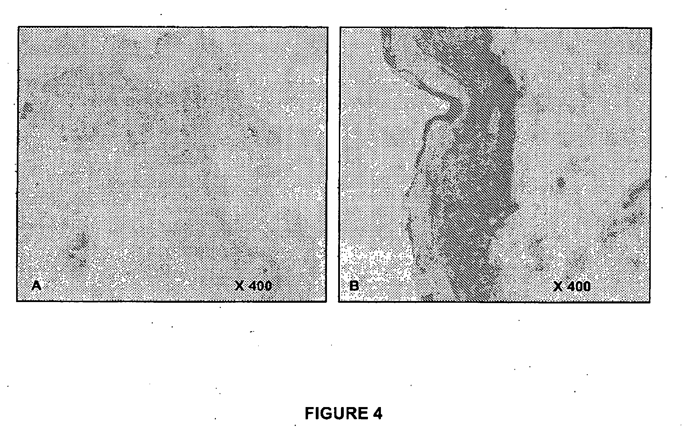

Ces résultats montrent que l'αβ-LBG brut à 30 µg/ml induit la synthèse des transcrits hBD-3 sur un temps court à 2 h avec une forte diminution de l'induction à 24 h (FI chutant de 17 à 3) selon une cinétique proche de celle de hBD-2. En ce qui concerne le peptide LL-37 son induction par l'αβ-LBG brut à 30 µg/ml est effective dès 2 h de traitement avec un FI de 6 qui reste stable jusqu'à 24 h.
➢L'analyse qualitative de l'expression peptidique par immuno-histochimie à partir des explants stimulés par voie systémique a permis de confirmer la stimulation de la production des peptides hBD-2 et hBD-3 dans les couches supérieures de l'épiderme après stimulation par l'αβ-LBG brut pendant 24h. La figure 4 annexée correspond aux photos immuno-histologiques.
➢ L'induction de l'expression du gène hBD-2 a été quantifiée au niveau des ARNm spécifiques transcrits par les cellules épithéliales cutanées au sein d'explants de peau après incubation topique d'αβ-LBG brut à 30 et 300 mg/ml en comparaison du témoin de stimulation, la toxine bactérienne SEB à 50 µg/ml.

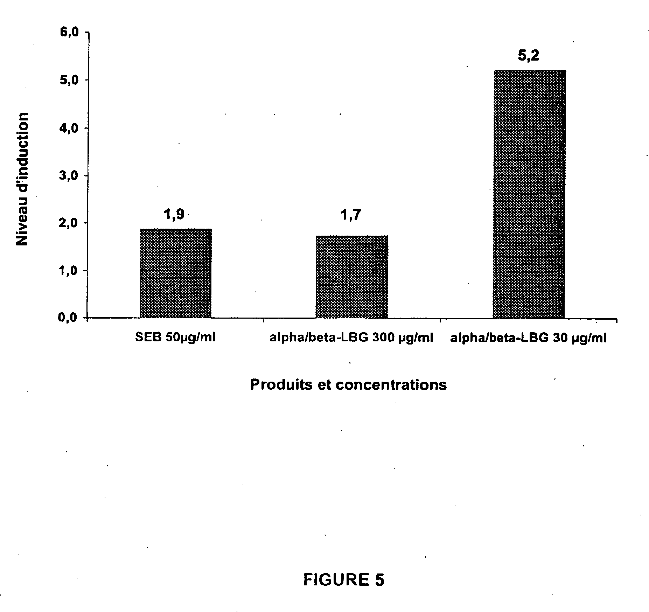

La figure 5 annexée illustre l'induction de l'expression du gène hBD-2 au sein d'un explant cutané par l'α,β-LBG brut à différentes concentrations par voie topique pendant 5 h : quantification de la synthèse des transcrits hBD-2 par RT-PCR quantitative.

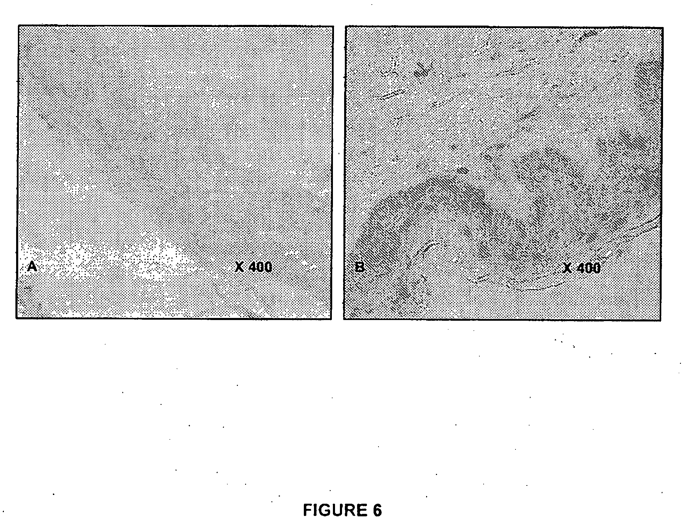

Ces résultats montrent que l'αβ-LBG sous sa forme brute est capable d'induire par voie topique la synthèse des transcrits hBD-2 au niveau de l'épithélium cutané dès la concentration de 30 µg/ml, FI de 5,2 et dans une moindre mesure à une concentration de 300 µg/ml, FI de 1,7. Pour comparaison, le SEB présente une activité du même ordre que celle de l'α,β-LBG (300 µg/ml) avec un FI de 1,9. De plus cette induction est effective dès 5 h d'incubation.
➢L'analyse qualitative de l'expression peptidique par immuno-histochimie à partir des explants stimulés par voie topique a permis de confirmer qualitativement la stimulation de la production des peptides hBD-2 et hBD-3 dans les couches supérieures de l'épiderme après stimulation par l'αβ-LBG. La figure 6 annexée correspond aux photos immuno-histologiques.

Le potentiel inducteur des isoformes de l'ester de laurate de butylglucoside pur à 90 %, séparées, α seul ou β seul, ou en mélange α,β-LBG (56 % α/44 % β) a été comparé sur explant cutané par voie systémique ou topique.
➢L'induction de l'expression du gène hBD-2 a été quantifiée au niveau des ARNm spécifiques transcrits par les cellules épithéliales cutanées au sein d'explants de peau incubés de manière systémique en présence de TNFα à 500 ng/ml, d'IL1-β à 100 ng/ml, d'αβ-LBG pur, d'α-LBG ou de β-LBG pur à 3 µg/ml pour une durée de traitement de 2 h ou de 15 h.

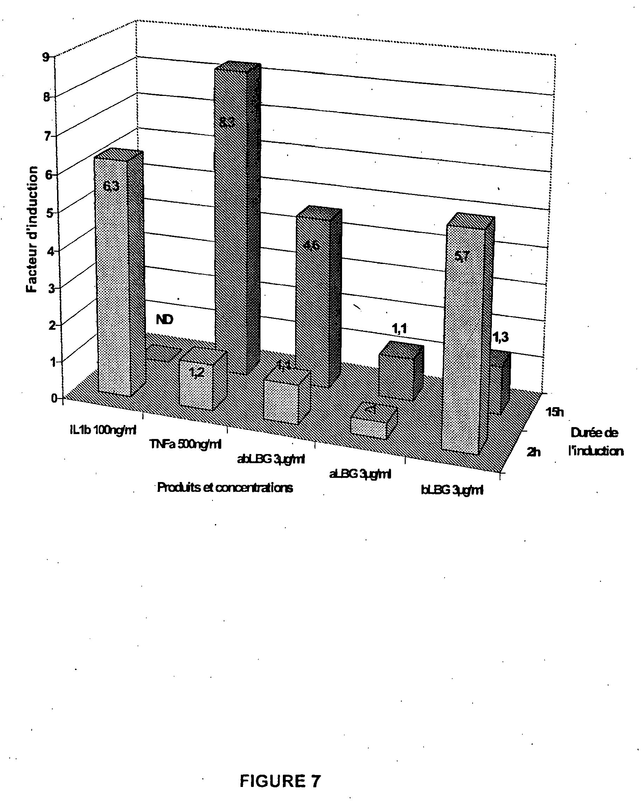

La figure 7 annexée illustre la cinétique d'induction par voie systémique pendant 2 et 15 h de l'expression du gène hBD-2 au sein d'un explant cutané par l'α,β-LBG pur à 3 µg/ml : Comparaison des formes αβ (ab), α (a) et β (b) LBG ; quantification de la synthèse des transcrits hBD-2.

A une concentration donnée identique, 3 µg/ml, les isoformes en mélange αβ-LBG pur à 3 µg/ml présentent un potentiel inducteur significatif surtout après une durée de traitement longue. Après séparation, l'isoforme β-LBG pure à 3 µg/ml, isolée à plus de 95 % vis-à-vis de l'isoforme α-LBG est plus active que le mélange dès 2 h de traitement (FI de 5,7 contre 1,1). A l'inverse, l'isoforme α-LBG pure ne présente pas d'activité à cette même concentration sur l'induction de l'expression du gène hBD-2 dans les cellules épithéliales cutanées même après un traitement long de 15 h.

Dans nos conditions expérimentales, ces résultats montrent que l'isoforme β-LBG serait à l'origine de l'activité pharmacologique inductrice de l'expression du gène hBD-2.

Par voie topique
➢L'induction de l'expression du gène hBD-2 a été quantifiée au niveau des ARNm spécifiques transcrits par les cellules épithéliales cutanées au sein d'explants de peau incubés de manière topique en présence *d'*αβ*-LBG pur,* d'α-LBG ou de β-LBG pur à 3 ou 30 µg/ml pour une durée de traitement de 18 h.

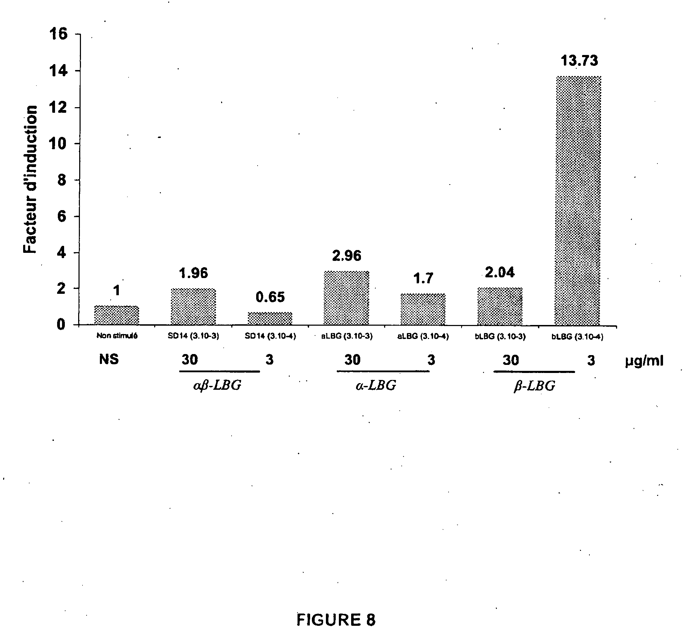

La figure 8 annexée illustre l'induction de l'expression du gène hBD-2 au sein d'un explant cutané par voie topique après 18 h de traitement par l'α,β-LBG pur à 3 ou 30 µg/ml : Comparaison des formes αβ, α et β-LBG; quantification de la synthèse des transcrits hBD-2 par RT-PCR en temps réel.

Après 18 h de traitement topique par deux concentrations données identiques, 30 et 3 µg/ml, le mélange αβ-LBG ainsi que les isoformes α eβ-LBG purs isolés induisent une expression du gène de hBD-2 de même ordre au niveau de la transcription (FI entre 1,96 et 2,96). Lorsque la concentration est abaissée à 3 µg/ml, l'isoforme β-LBG pur (FI de 13,7), est nettement plus active que le mélange αβ-LBG pur et que l'isoforme α-LBG pure

Dans nos conditions expérimentales, ces résultats confirment par voie topique ceux obtenus par voie systémique. L'isoforme β-LBG serait à l'origine de l'activité pharmacologique inductrice de l'expression du gène hBD-2.

Le dénombrement de la flore bactérienne adhérente exogène par unité de surface de peau a été effectué selon deux méthodes :
- par dénombrement bactérien sur milieu solide
- par comptage de la radioactivité émise (la souche contaminante étant marquée au H³) est effectuée.

La comparaison des résultats a permis de montrer qu'il n'existait pas de différence significative entre les deux méthodes. L'analyse par dénombrement a donc été conservée pour le test d'adhésion de S. *aureus* sur tissu cutané.

Après une application topique de 5 h et plusieurs rinçages de la surface épidermique d'un explant cutané, l'effet du traitement par l'α,β-LBG brut sur l'adhésion de *S.aureus* sur ce même explant a été quantifié par le dénombrement des germes adhérents à l'issue d'une mise en contact de 2 h.

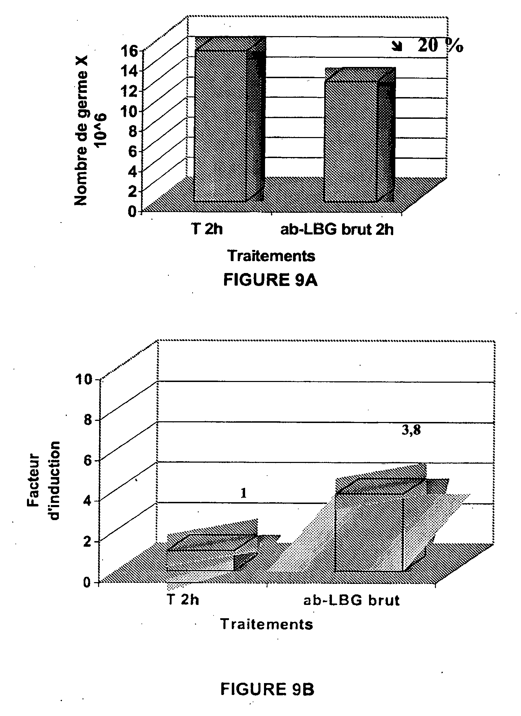

La figure 9 annexée représente l'activité de l'α,β-LBG brut appliqué en topique à une dose de 30 µg/ml sur un explant cutané sur l'adhésion de S. *aureus* (A) corrélé à l'induction de l'expression du gène hBD-2 (B) au niveau de cet explant en comparaison avec le tissu du même donneur non stimulé (quantification des ARNms spécifiques par PRC en temps réel).

Les résultats montrent un effet régulateur significatif du traitement par l'α,β-LBG brut sur le nombre de germes pathogènes adhérents par rapport au témoin non traité, avec une diminution de ce nombre équivalent à une baisse de 20 % de l'adhésion de S. *aureus* (figure 9A).

Parallèlement, l'induction de l'expression du gène hBD-2 a été vérifiée après 5 h de traitement par l'α,β-LBG brut. La persistance de cette état d'induction a été démontrée après l'étape d'adhésion (2 h) de S. *aureus* avec un FI de 3,8 par rapport au témoin non traité mais en tout point identique en ce qui concerne les autres étapes telles que l'adhésion (figure 9B).

Sans avoir directement été démontré il serait envisageable que l'induction des bêta-défensines puisse être corrélée à l'effet de l'α,β-LBG brut sur la fonctionnalité du tissu épithélial cutané et en particulier sur la ré-équilibration de la flore cutanée. Une inhibition spécifique de cette induction après traitement pourrait permettre de vérifier cette hypothèse.

Bien qu'il ait été vérifié qu'aux concentrations utilisées l'α,β-LBG brut ne présentait ni activité microbicide directe ni effet sur l'adhésion de *S. aureus,* des expérimentations ont été menées afin de vérifier la nature indirecte de l'action de l'α,β-LBG sur l'interaction entre S. *aureus* et épithélium cutané. Pour cela, le compartiment de traitement du tissu par l'α,β-LBG et le compartiment d'interaction entre l'épithélium cutané et le germe pathogène ont été séparés, le premier ayant lieu côté profond de l'épiderme (voie systémique) et le second à la surface épidermique (voie topique).

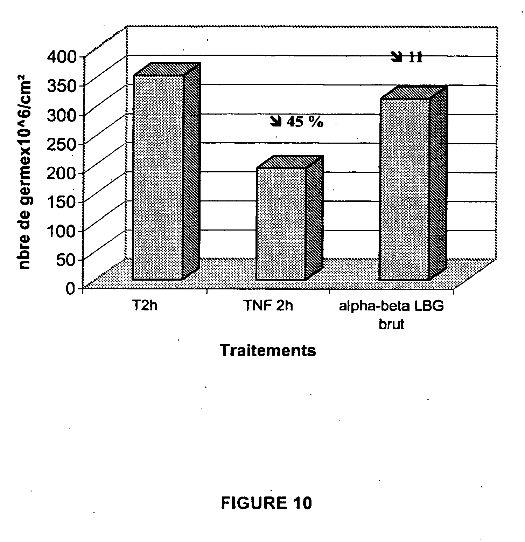

La figure 10 annexée illustre l'activité indirecte de l'α,β-LBG brut appliqué en systémique à une dose de 30 µg/ml sur un explant cutané, sur l'adhésion de S. *aureus* ou en comparaison avec le tissu du même donneur non stimulé ou traité par le TNFα (quantification des ARNm spécifiques par PRC en temps réel.

Les résultats de dénombrement montrent que sans contact direct avec la surface sur laquelle a lieu le contact ultérieur des micro-organismes, l'α,β-LBG est capable de réguler l'adhésion des germes sur l'explant (diminution de 11 % par rapport au témoin non stimulé), via la régulation à distance de l'expression de molécules antimicrobiennes épithéliales. Parmi les candidats moléculaires potentiels, les bêta-défensines stimulées par l'α,β-LBG ou le TNF-α pourraient représenter ce lien. Ainsi, la cytokine pro-inflammatoire, le TNF-α, entraîne une forte chute de l'adhésion de S. aureus corrélée à une forte induction des bêta-défensines épithéliales.

Après application topique et plusieurs rinçages de la surface épidermique des explants cutanés, l'effet du traitement par les isoformes de l'ester de laurate de butylglucoside pur à 90 %, séparées, α seul, β seul, ou en mélange α,β-LBG (56 % α/44 % β) sur l'adhésion de S. *aureus* sur l'épiderme a été comparé après quantification des germes adhérents par dénombrement.

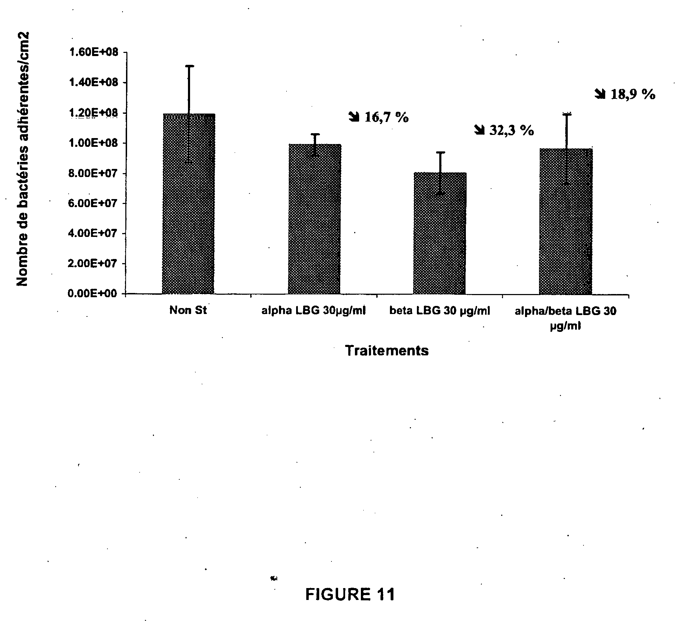

La figure 11 annexée illustre l'activité des différentes isoformes de LBG pur à 90 % appliquées en topique à une dose de 30 µg/ml sur un explant cutané sur la régulation de l'adhésion de *S*. *aureus :* comparaison des formes α et β-LBG et du mélange α/β.

Les résultats montrent un effet régulateur du traitement par les différentes préparations pures de LBG sur le nombre de germes pathogènes adhérents par rapport au témoin non traité, avec une diminution de ce nombre équivalente à une baisse moyenne de l'ordre de 19 % de l'adhésion de *S*. *aureus* pour le mélange αβ-LBG pur. Pour comparaison, l'isoforme β-LBG pur est en moyenne plus actif sur la limitation de l'adhésion de *S. aureus* que l'isoforme α-LBG.

Il est par ailleurs possible d'observer la colonisation de l'explant par le germe exogène *(S. aureus)* après l'étape d'adhésion pendant 24 h. Il est intéressant de noter que l'effet d'un traitement systémique par l'α,β-LBG brut ralentit la multiplication du germe pathogène et favorise à l'inverse le développement d'un germe commensal endogène *Staphylococcus epidermidis (S. epidermidis*) (présent sur l'explant au départ). L'effet inverse est observé avec le traitement par le TNF-α, cytokine pro-inflammatoire, qui après avoir entraîné une forte chute de l'adhésion de *S. aureus* (Cf.III.4.2) ne permet pas une limitation de la croissance du germe pathogène sur l'explant et ceci au dépend du germe commensal *S*. *epidermidis.*

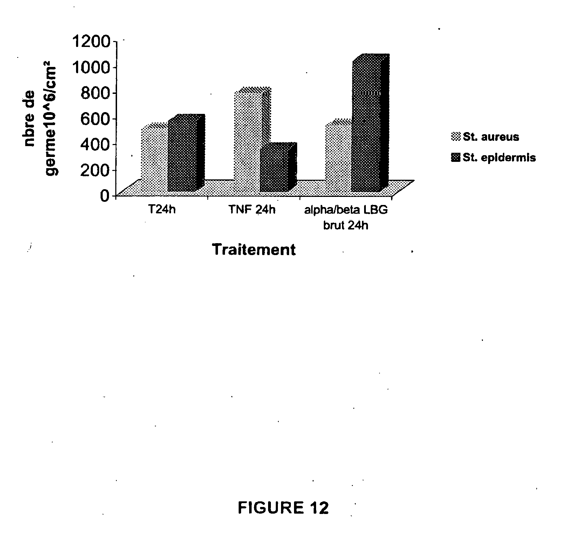

La figure 12 annexée illustre la comparaison de la colonisation d'une souche contaminante, *S. aureus,* avec celle d'une souche commensale, *S. epidermis.*

Ces deux traitements semblent donc perturber l'écosystème originel. Cependant, le déséquilibre induit par ces stimulations est en faveur de la souche commensale dans le cas de l'α,β-LBG.

L'ester de laurate, αβ-LBG brut à 30 µg/ml pur à 48,6 % dont 90 % de la forme α-LBG et 10 % de forme β-LBG a été testé sur explant gingival par voie systémique uniquement, l'orientation du tissu étant difficilement réalisable contrairement aux explants cutanés après un traitement de 5 h.
➢ L'analyse qualitative de l'expression peptidique à partir des explants stimulés par voie systémique a été réalisée par immuno-histochimie avec un anticorps dirigé contre le peptide hBD-3.

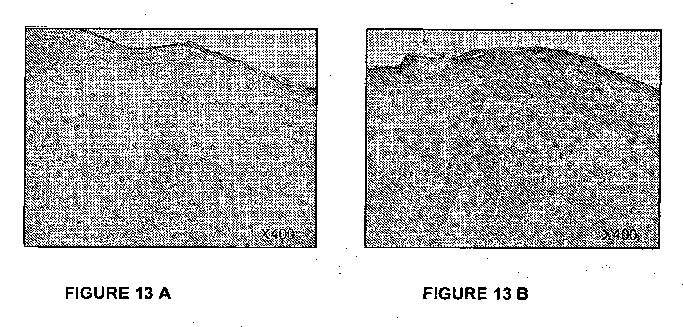

La figure 13 annexée illustre l'activité de l'αβ-LBG pur sur l'induction de l'expression du peptide hBD-3 au niveau de l'épithélium gingival d'un donneur sain après 5 h de traitement en systémique. Localisation du marquage spécifique hBD-3 au sein de l'épithélium traité par l'actif en systémique pendant 5 h (B) et comparaison avec le tissu du même donneur non stimulé (A).

Après révélation, la comparaison des marquages a permis de démontrer l'induction de la production du peptide hBD-3 dans les couches supérieures de l'épithélium gingival après traitement du tissu par l'αβ-LBG.

Il est à noter, que pour l'épithélium gingival, l'expression des peptides antimicrobiens épithéliaux varie fortement selon le donneur non seulement du fait de la richesse de la flore présente au sein de l'écosystème buccal mais aussi du fait de l'état physiologique de l'épithélium (inflammation gingivale, pathologie parodontale...). L'utilisation d'un modèle tissulaire épithélial reconstruit à partir de cellules épithéliales gingivales primaires issues d'un même donneur, présente un intérêt majeur dans l'évaluation de principes actifs.

L'ester de laurate, αβ-LBG brut à 30 µg/ml pur à 48,6 % dont 90 % de la forme α-LBG et 10 % de forme β-LBG a été testé sur épithélium gingival reconstruite (SkinEthic Laboratories) après un traitement de 18 h par voie topique ou systémique.
➢ L'induction de l'expression du gène hBD-3 a été quantifiée au niveau des ARNm spécifiques transcrits par les cellules épithéliales gingivales au sein de l'épithélium reconstruit.

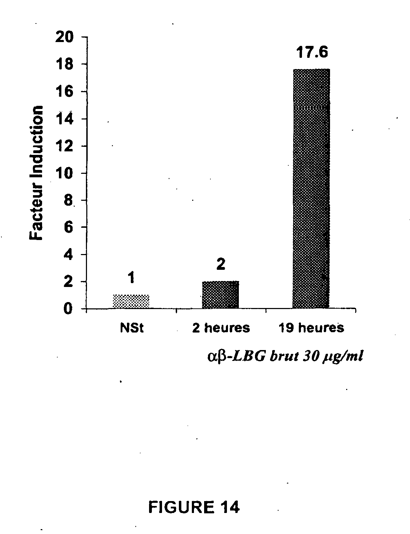

La figure 14 annexée illustre la cinétique d'activité de l'αβ-LBG pur sur l'induction de l'expression du gène codant pour hBD-3 au niveau d'un épithélium gingival reconstruit (SkinEthic Laboratories) : quantification des ARNm spécifiques par PCR en temps réel.

La quantification des transcrits hBD-3 démontre la capacité de l'αβ-LBG sous sa forme brute à induire la synthèse des transcrits hBD-3 au niveau de l'épithélium cutané par voie topique à la concentration de 30 µg/ml par rapport à un témoin non traité (Nst) : à partir de 2 h de traitement (FI de 2) et jusqu'à 19 h d'incubation (FI de 17,6).
➢L'analyse qualitative de l'expression peptidique par immuno-histochimie à partir d'épithélium gingival reconstruit traité par l'αβ-LBG brut en comparaison d'un échantillon non traité, a permis de confirmer la production du peptide hBD-3 par les cellules de différentes strates de l'épithélium gingival reconstruit et en particulier localiser son accumulation dans les couches les plus superficielles.

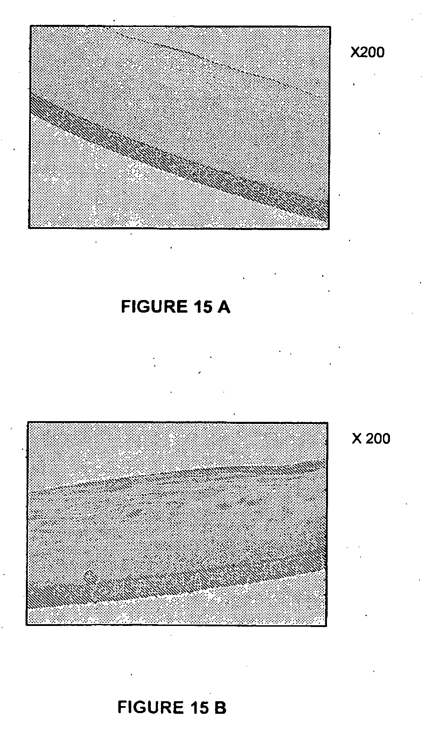

La figure 15 annexée illustre l'activité des αβ-LBG purs sur l'induction de l'expression du peptide hBD-3 au niveau d'un épithélium gingival reconstruit (SkinEthic laboratories) : localisation du marquage spécifique de l'épithélium traité par l'actif en systémique pendant 1 h (B) et comparaison avec le tissu du même donneur non stimulé (A).

Cependant, l'apparition de ce marquage étant très rapide il est probable que le peptide ne soit pas issu d'une néo-synthèse du peptide.

En conclusion, l'ensemble des résultats démontrent l'activité pharmacologique des alkyles mono- ou di-esters d'α,β-butylglucoside sur l'induction de l'expression des gènes hBD-2, hBD-3 et LL-37 sur des modèles cellulaires, et tissulaires par voie topique ou systémique aussi bien au niveau de l'épithélium cutané que gingival.

Par ailleurs les données de fonctionnalité *ex vivo* sur l'adhésion d'un germe pathogène exogène permettent de mettre en évidence une activité régulatrice de ces molécules sur la flore cutanée et en particulier sur la ré-équilibration de cet écosystème vers une flore commensale.

Le lien direct entre stimulation de l'expression des peptides antimicrobiens et régulation de la flore reste à établir mais il est tout à fait envisageable du fait de la corrélation entre régulation de la flore induction de l'expression des gènes de ces peptides au niveau épithélial en présence d'α,β-LBG.

Enfin, l'analyse de l'activité des différentes isoformes a permis de mettre en évidence la supériorité de l'isoforme β-LBG vis-à-vis de l'isoforme α-LBG aussi bien d'un point de vue pharmacologique *in vitro* que d'un point de vue fonctionnalité *ex vivo.*

On indiquera ci-après un certain nombre d'exemples de compositions selon l'invention. En fonction de leur pureté, deux types d'APG sont utilisés.

Les premiers sont à 70 % de laurate de butylglucoside et comme ils sont très visqueux ils sont dilués au demi avec de l'octyl palmitate. L'ensemble constitue un noyau à 35 % environ de LBG.

Les seconds produits, plus purifiés, sont à 85 % voire au-delà et sont utilisés tels quels.

### EXEMPLE 1

| Pâte dentifrice : | |
|---|---|
| Octyl palmitate /LBG | 1 à 5% ou LBG 0,5 à 2,5 % |
| Phosphate dicalcique | 60% |
| Glycérine | 15% |
| Gomme adragante / Xanthane | 1% |
| Parfum | 1% |
| Eau | QSP100 |
| Agent moussant type sodium laurylsulfate | 1% |

### EXEMPLE 2

| Gel antimicrobien : | |
|---|---|
| Octyl palmitate /LBG | 1 à 5% ou LBG 0.5 à 2,5 % |
| Natrosol HHX 250 | 1,5% |
| Chlorhexidine digluconate | 0,2% |
| alcool | 15 % à 20 % vol |
| Eau déminéralisée | qsp100 |

### EXEMPLE 3

| Cold cream : | |
|---|---|
| LBG noyau | 1 à 5% |
| Cires végétales | 30% |
| Huile d'amande douce | 50% |
| Borate de sodium | 0,5 % |
| Phénoxyéthanol | 0,8% |
| Eau | qsp100 |

### EXEMPLE 4

| Dépilatoire : | |
|---|---|
| LBG noyau | 5% |
| Gélatine | 2% |
| Thioglycoltate de calcium | qs |
| pH 11,5 - 12 Eau | qsp100 |

### EXEMPLE 5

| Lait antiseptique : | |
|---|---|
| | |
| LBG | 2 à 5% |
| Huile minérale | 10% |
| Huile de coprah | 15% |
| Ester sorbitane | 20% |
| Conservateurs | 1 % |
| Eau | qsp100 |
| Polymère acrylique pour stabiliser | qsp pH 7 |

### EXEMPLE 6

| Crème pour les mains : | |
|---|---|
| LBG noyau | 2 à 5% |
| Huile de vaseline | |
| huile de silicone | |
| glycérine | |
| stéarate de triéthanolamine | |
| BHT | |
| Parahydroxybenzoate de méthyle | |
| Parfum | |
| Eau déminéralisée | qsp 100 |

### EXEMPLE 7

| Savon liquide : | |
|---|---|
| LBG noyau | 5% |
| Huile de palme | 80% |
| Sodium hydroxyde | 12% |
| Glycérine | 10% |
| Parfum | 0.5% |
| Conservateur | 1% |

### REFERENCES BIBLIOGRAPHIQUES

Brevet WO 93/04185
Chung WO., Hansen SR., Rao D., Dale BA. Protease-Activated Receptor signaling increases epithelial antimicrobial peptide expression. J.Immunol. 2004, 173: 5165-5170.
Chung WO., Dale BA. Innate immune response of oral and foreskin keratinocytes: utilization of different pathways by various bacterial species. Infect. Immun. 2004, 72: 352-358.
Frohm M., Agerberth B., Ahangari G., Stahle-Bäckdahl M., Lidén S., Wigzell H., Gudmundsson GH. The expression of the gene encoding for antibacterial peptide LL-37 is induced in human keratinocytes during inflammatory disorders. J. Biol. Chem. 1997, 272: 15258-15263.
Ganz T. Defensins and host defense, Science, 1999, 286, 420-421.
Gordon S. Pattern Recognition Receptors: Doubling up for the innate immune response. Cell 2000. 2002, 111: 927-930.
Kao CY., Chen Y., Thai P., Wachi S., Huang F., Kim C., Harper RW., Wu R. IL-17 markedly up-regulates β-Defensin-2 expression in human airway epithelium via JAK and NF- B signaling pathways. J.Immunol. 2004, 173: 3482-3491.
Koczulla R., von Degenfeld G., Kupatt C., Krötz F., Zahler S., Gloe T., Issbrücker K., Unterberger P., Zaiou M., ..Bals R. An angiogenic role for the human peptide antibiotic LL-37/hCAP18. J. Clin. Invest. 2003, 111: 1665-1672.
Koczulla A.R. et Bals R. Antimicrobial peptides: current status and therapeutic potential, Drugs, 2003, 63, 389-406.
Krisanaprakornkit S., Kimball JR., Dale BA. Regulation of Human β-Defensin-2 in gingival epithelial cells: the involvement of Mitogen-Activated Protein Kinase pathways, but not the NF-κB transcription factor family. J.Immunol. 2002, 168: 316-324.
Liu A.Y., Destoumieux D., Wong A.V., Park C.H., Valore E.V., Liu L. et Ganz T. Human beta-defensin-2 production in keratinocytes is regulated by interleukin-1, bacteria, and the state of differentiation, J. Invest Dermatol., 2002, 118, 275-281.
Ong Y.P., Ohtake T., Brandt C., Strickland I., Boguniewicz M., Ganz T., Gallo R.L., and Leung D.Y.M., Endogenous antimicrobial peptides and skin infections in atopic dermatitis. N Eng J Med, 347, No. 15. 2002.
Presland RB., Dale BA. Epithelial structural proteins of skin and oral cavity : function in health and disease. Crit. Rev. Oral Biol. Med. 2000, 11: 383-408. Scott M. G., Davidson D. J., Gold M. R., Bowdish D., Hancock R. E. W., The human antimicrobial peptide LL-37 is a multifunctional modulator of innate immune responses. The Journal of Immunology, 2002, 169: 3883-3891.
Vandesompele J., De Preter K., Pattyn F., Poppe B., Van Roy N., De Paepe A. et Speleman F. Accurate normalization of real-time quantitative RT-PCR data by geometric averaging of multiple internal control genes, Genome Biol., 2002, 3, RESEARCH I-0034.
Yang D., Biragyn A., Hoover D.M., Lubkowski J., Oppenheim J.J. Multiple roles of antimicrobial defensins, cathelicidins, and eosinophil-derived neurotoxin in host defense, Annu. Rev. Immunol., 2004, 22, 181-215.
Zaiou M., Nizet V., Gallo RL. Antimicrobial and protease inhibitory functions of human Cathelicidin (hCAP18/LL-37). J Invest. Dermatol. 2003, 120: 810-816.
Zanetti M. Cathelicidins, multifunctional peptides of the innate immunity, Journal of Leukocyte Biology, 2004, 75.

## Revendications

1. Esters d'alkylglucoside comme principes actifs provoquant un rééquilibrage de la flore commensale de la peau et des muqueuses et/ou une diminution de l'adhésion des microorganismes pathogènes sur le tissu épithélial par induction de l'expression de peptides antimicrobiens endogènes, pour son utilisation dans une composition topique dermatologique ou dermocosmétologique destinée au traitement préventif ou curatif des affections microbiennes.

2. Esters selon la revendication 1, **caractérisés en ce que** les peptides antimicrobiens endogènes appartiennent à la famille des β-défensines et des cathélicidines.

3. Esters selon l'une des revendications 1 ou 2, **caractérisés en ce que** les peptides antimicrobiens endogènes de la famille des β-défensines sont alors HBD-2 et HBD-3.

4. Esters selon l'une des revendications 1 à 3, **caractérisés en ce que** le peptide antimicrobien endogène de la famille des cathélicidines est le LL-37.

5. Esters selon l'une des revendications 1 à 4, **caractérisés en ce que** la composition topique est destinée à favoriser l'homéostasie épithéliale pour le traitement de la dermatite atopique.

6. Esters selon l'une des revendications 1 à 5, **caractérisés en ce que** la composition est destinée à favoriser l'homéostasie épithéliale pour le traitement d'affections ophtalmiques.

7. Esters selon l'une des revendications 1 à 6, **caractérisés en ce que** la composition est destinée à favoriser l'homéostasie épithéliale pour le traitement d'affections gingivales.

8. Esters selon l'une des revendications 1 à 7, **caractérisés en ce que** la composition est destinée à favoriser l'homéostasie épithéliale dans le traitement d'affections vaginales.

9. Esters selon l'une des revendications 1 à 8, **caractérisés en ce que** l'ester d'alkylglucoside est un C₈-C₂₀-alkyle mono- ou di-ester de C₁-C₆-alkylglucoside.

10. Esters selon l'une des revendications 1 à 9, **caractérisés en ce que** l'ester d'alkylglucoside est un C₈-C₂₀-alkyle mono- ou di-ester de butylglucoside.

11. Esters selon l'une des revendications 1 à 10, **caractérisés en ce que** l'ester d'alkylglucoside est un mono- ou di-caprylate, -caprate, -laurate, -palmitate, - myristate, -stéarate, -cocoate d'α-butylglucoside.

12. Esters selon l'une des revendications 1 à 11, **caractérisés en ce que** l'ester d'alkylglucoside est le laurate d'α-butylglucoside, de préférence dilué dans le palmitate d'octyle.

## Patentansprüche

1. Ester von Alkylglucosiden als Wirkstoffe, die eine Wiederherstellung des Gleichgewichts der kommensalen Flora der Haut und der Schleimhäute bewirken und/oder eine Verminderung der Adhäsion von pathogenen Mikroorganismen auf dem Epithelgewebe durch Induktion der Expression von endogenen antimikrobiellen Peptiden, zur Verwendung in einer dermatologischen oder dermokosmetologischen topischen Zusammensetzung, die zur präventiven oder heilenden Behandlung von mikrobiellen Erkrankungen bestimmt ist.

2. Ester nach Anspruch 1, **dadurch gekennzeichnet, dass** die endogenen antimikrobiellen Peptide zur Familie der β-Defensine und der Cathelicidine gehören.

3. Ester nach einem der Ansprüche 1 oder 2, **dadurch gekennzeichnet, dass** die endogenen antimikrobiellen Peptide der Familie der β-De-fensine HBD-2 und HBD-3 sind.

4. Ester nach einem der Ansprüche 1 bis 3, **dadurch gekennzeichnet, dass** das endogenen antimikrobielle Peptid der Familie der Cathelicidine LL-37 ist.

5. Ester nach einem der Ansprüche 1 bis 4, **dadurch gekennzeichnet, dass** die topische Zusammensetzung zur Begünstigung der epithelialen Homöostase für die Behandlung der atopischen Dermatitis bestimmt ist.

6. Ester nach einem der Ansprüche 1 bis 5, **dadurch gekennzeichnet, dass** die Zusammensetzung zur Begünstigung der epithelialen Homöostase für die Behandlung von ophthalmologischen Erkrankungen bestimmt ist.

7. Ester nach einem der Ansprüche 1 bis 6, **dadurch gekennzeichnet, dass** die Zusammensetzung zur Begünstigung der epithelialen Homöostase für die Behandlung von gingivalen Erkrankungen bestimmt ist.

8. Ester nach einem der Ansprüche 1 bis 7, **dadurch gekennzeichnet, dass** die Zusammensetzung zur Begünstigung der epithelialen Homöostase bei der Behandlung von vaginalen Erkrankungen bestimmt ist.

9. Ester nach einem der Ansprüche 1 bis 8, **dadurch gekennzeichnet, dass** der Alkylglucosid-Ester ein C₈-C₂₀-Alkyl-Mono- oder -Di-Ester von C₁-C₆-Alkylglucosid ist.

10. Ester nach einem der Ansprüche 1 bis 9, **dadurch gekennzeichnet, dass** der Alkylglucosid-Ester ein C₈-C₂₀-Alkyl-Mono- oder -Di-Ester von Butylglucosid ist.

11. Ester nach einem der Ansprüche 1 bis 10, **dadurch gekennzeichnet, dass** der Alkylglucosid-Ester ein Mono- oder Di-Caprylat, -Caprat, -Laurat, -Palmitat, -Myristat, -Stearat, -Cocoat von α-Butylglucosid ist.

12. Ester nach einem der Ansprüche 1 bis 11, **dadurch gekennzeichnet, dass** der Alkylglucosid-Ester ein α-Butylglucosid-Laurat ist, vorzugsweise verdünnt in Octylpalmitat.

## Claims

1. Alkyl glucoside esters as active substances inducing a rebalancing of the commensal flora of the skin and mucosa and/or a reduction of the adhesion of pathogenic micro-organisms on epithelial tissue by inducing the expression of endogenous antimicrobial peptides, for use in a dermatological or dermocosmetological topical composition for the preventive or curative treatment of microbial diseases.

2. Esters according to claim 1, **characterised in that** the endogenous antimicrobial peptides belong to the β-defensin and cathelicidin family.

3. Esters according to any of claims 1 or 2, **characterised in that** theendogenous antimicrobial peptides of the β-defensin family are HBD-2 and HBD-3.

4. Esters according to any of claims 1 to 3, **characterised in that** the antimicrobial peptide of the cathelicidin family is LL-37.

5. Esters according to any of claims 1 to 4, **characterised in that** the topical composition is intended to promote epithelial homeostasis for treating atopic dermatitis.

6. Esters according to any of claims 1 to 5, **characterised in that** the composition is intended to promote epithelial homeostasis for treating ophtalmic diseases.

7. Esters according to any of claims 1 to 6, **characterised in that** the composition is intended to promote epithelial homeostasis for treating gum diseases.

8. Esters according to any of claims 1 to 7, **characterised in that** the composition is intended to promote epithelial homeostasis for treating vaginal diseases.

9. Esters according to any of claims 1 to 8, **characterised in that** the alkyl glucoside ester is a C₈-C₂₀-alkyl mono- or di-ester of C₁-C₆-alkyl glucoside.

10. Esters according to any of claims 1 to 9, **characterised in that** the alkyl glucoside is a C₈-C₂₀-alkyl mono- or di-ester of butyl glucoside.

11. Esters according to any of claims 1 to 10, **characterised in that** the alkyl glucoside ester is an α-butyl glucoside mono- or di-caprylate, -caprate,-laurate, -palmitate, -myristate, -stearate, -cocoate.

12. Esters according to any of claims 1 to 11, **characterised in that** the alkyl glucoside ester is α-butyl glucoside laurate, preferably diluted in octyl palmitate.
